# EUROPEAN PATENT SPECIFICATION

(11) **EP 2 934 504 B1**
(45) Date of publication and mention of the grant of the patent: **25.04.2018**
(21) Application number: 13821686.6
(22) Date of filing: 19.12.2013
(51) Int. Cl.: A61K 31/198, A61K 9/20, A61K 9/48, A61K 31/7004, A61P 5/14, A61P 17/00

(54) **A PHARMACEUTICAL FORMULATION COMPRISING INOSITOL AND SELENIUM, PREPARATION AND USE THEREOF**
PHARMAZEUTISCHE FORMULIERUNG MIT INOSITOL UND SELEN, HERSTELLUNG UND VERWENDUNG DAVON
FORMULATION PHARMACEUTIQUE COMPRENANT DE L'INOSITOL ET DU SÉLÉNIUM, SA PRÉPARATION ET SON UTILISATION

(30) Priority: 20.12.2012 IT FI20120288
(43) Date of publication of application: 28.10.2015
(73) Proprietor: LO. LI. Pharma S.r.l., 00156 Roma (IT)
(72) Inventor: UNFER, Vittorio, 00155 Roma (IT)
(74) Representative: Brighenti, Livio
(86) International application number: PCT/EP2013/077422
(87) International publication number: WO 2014/096209

(56) References cited:
- US-A1- 2002 182 196
- US-A1- 2008 114 065
- MAZOKOPAKIS, E.E. ET AL.: "Effects of 12 months treatment with L-selenomethionine on serum anti-TPO levels in patients with Hashimoto's thyroiditis", THYROID, vol. 17, no. 7, 2007, pages 609-612, XP002696021,
- TOULIS, K.A. ET AL.: "Selenium supplementation in the treatment of Hashimoto's thyroiditis: a systemic review and meta-.analysis", THYROID, vol. 20, no. 10, 2010, pages 1163-1173, XP002696022,
- A. D. ANASTASILAKIS ET AL: "Selenomethionine treatment in patients with autoimmune thyroiditis: a prospective, quasi-randomised trial", INTERNATIONAL JOURNAL OF CLINICAL PRACTICE, vol. 66, no. 4, 1 April 2012 (2012-04-01), pages 378-383, XP055060844, ISSN: 1368-5031, DOI: 10.1111/j.1742-1241.2011.02879.x
- CARLOMAGNO, G. ET AL.: "Myo-inositol in a new pharmaceutical form: a step forward to a new clinical use", EXPERT OPINION ON DRUG DELIVERY, vol. 9, no. 3, March 2012 (2012-03), pages 267-271, XP009169088,

## Description

### Field of the Invention

The present invention relates to the field of formulations containing inositol.

### State of the Art

If the importance of an adequate iodine intake to the body through the use of iodised salt is known and highlighted, the same cannot be said for another equally important element for thyroid function: selenium; selenium is, however, vitally important as this element has defensive and regulatory functions in our body.

Selenium is, in fact, critical to the functioning of certain enzymes, precisely called seleno- proteins, which, without this element, are not able to function properly, and the major enzymes for whose functioning the presence of selenium is required exert their action in thyroid metabolism.

A confirmation of the close link between the thyroid and selenium is given by thyroid peroxidase (TPO), the key enzyme for the synthesis of thyroglobulin and thyroid hormones.

From the chemical reactions required for the synthesis of thyroid hormones, in which thyroid peroxidase intervenes, however, free radicals are generated that would be dangerous and harmful if a defence system aimed at protecting the thyroid cell from oxidative damage was not active. This intra-thyroid defence system is represented, in large part, precisely by the selenium-dependent enzyme glutathione peroxidase. Other studies have also shown that low blood levels of selenium correspond to an increase of oxidative stress and thyroid tissue damage, with reduced production of thyroid hormones resulting in hypothyroidism.

It is, therefore, likely that a deficiency of selenium may trigger and maintain an autoimmune thyroiditis in patients predisposed to the development of the disease.

Therefore, selenium supplementation could have a great impact as the chronic autoimmune thyroiditis is among the most common endocrine disorders, affecting approximately 10% of female and 2% of male population. In addition, autoimmune thyroiditis is progressively increasing and represents the most common cause of hypothyroidism (50-80% of cases).

It has been demonstrated that supplementation with Se is capable of modifying the expression of HLA-DR, therefore modulating the immune response. In particular, it has been shown that in patients on L-T4 replacement therapy treated with Sodium Selenite 200g/d (2.53 mol/d) a 36% reduction of anti-TPO hormones versus the placebo group was observed, the additional analysis of a subgroup having baseline levels anti-TPO greater than 1200 IU/ml reported a 60% reduction (46).

In particular, a complete normalisation of antibody levels was observed in 25% of cases; this proportion reached 43% during follow-up (47).

Comparable results were obtained by administering Se-methionine to patients with AIT (48).

Inositol is a crucial element for the proper functioning of a myriad of biological processes; this polyalcohol is in fact the crucial part of the wider signal transduction system. Some examples of key hormones whose functioning is linked to the presence of myo-inositol are insulin and FSH.

In fact, thanks to these features, inositol has already been used in clinic for the treatment of PCOS and to improve the outcome of assisted reproduction procedures.

In similar biological processes, myo-inositol is the second messenger of TSH and T3.

Data in the literature have demonstrated that the myo-inositol dependent TSH signaling is responsible for the regulation of the synthesis of the hormone (T3), and the mobilisation of iodine. In particular, it has been observed that alterations of myo-inositol signaling induce a resistance to TSH; in fact, patients with a mutation that reduces the efficiency of signaling need to have TSH values 10 times the reference values in order to have comparable values of T3.

Conversely, in a study conducted on animal models, it has been demonstrated that the administration of a diet enriched in myo-inositol induced a reduction of T3 levels.

In the light of the aforegoing, there is a clear interest to develop a formulation capable of restoring and/or maintaining the euthyroid state in subjects with hypothyroidism normalising the levels of TSH, T3 and T4, and also capable of reducing the concentration of anti-peroxidase antibodies (anti-TPO) and anti-thyroglobulin (anti-TG) in case of hypothyroidism, autoimmune thyroiditis or Hashimoto's thyroiditis.

### Brief description of the figures

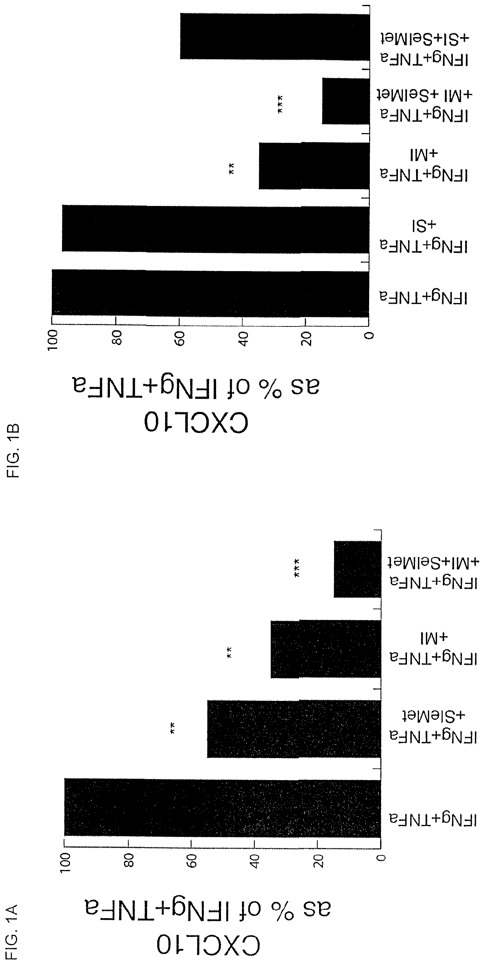

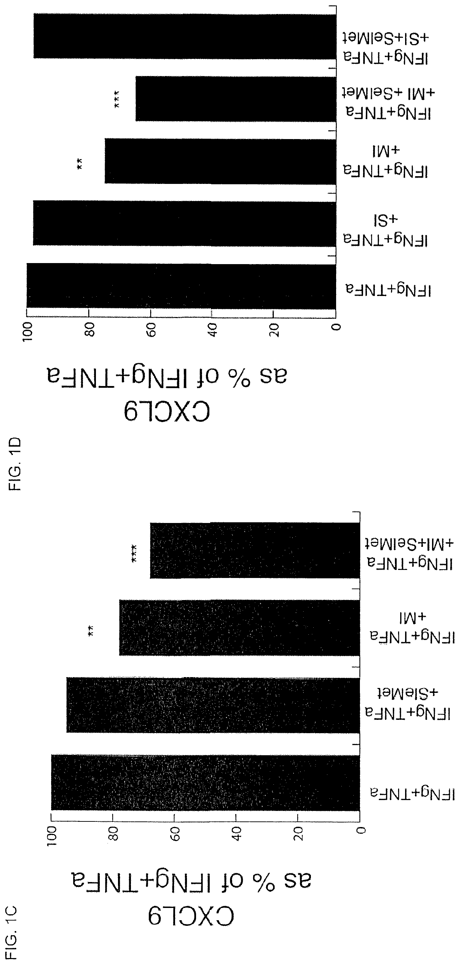

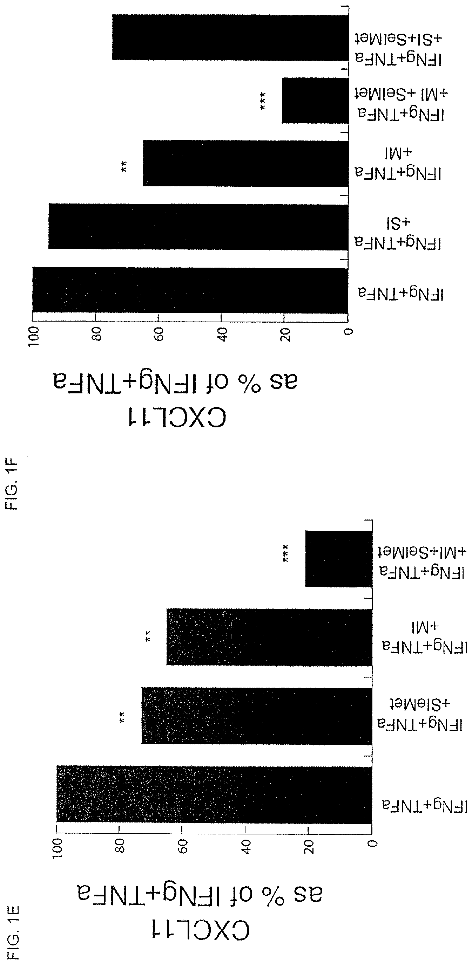

Figures 1A to 1F show the effect of the formulations according to the invention in tests on autoimmune disease.

### Summary of the Invention

Pharmaceutical formulations comprising Se-methionine in amounts between 10 and 400 ug and inositol in amounts between 100 and 4,000 mg.

### Detailed Description of the Invention

The present invention allows solving the aforementioned problem thanks to formulations comprising from 100 to 4,000 mg of inositol and from 10 to 400 ug (micrograms) of selenium methionine.

According to the disclosure inositol means any one of its nine different stereoisomers: myo-inositol, D-chiro-inositol, L-chiro-inositol, scyllo-inositol, muco-inositol, neo-inositol, allo-inositol, epi-inositol and cis-inositol, or mixtures thereof; preferred are myo-inositol, D-chiro-inositol, or mixtures thereof.

Equally preferred are mixture consisting of myo-inositol and D-chiro-inositol.

In one embodiment according to the invention, the inositol as defined above is micro-encapsulated.

The pharmaceutical formulations according to the invention are normally suitable for oral administration, for example in the form of: powder, tablets, soft capsules, hard capsules, solution and the like.

The formulations are prepared using the excipients commonly used in pharmacopoeia for similar purposes such as: gelatine, glycerol, cellulose, methyl cellulose, fatty acids, surfactants and emulsifiers that are processed according to known techniques.

For the preparation of formulations according to the invention, the normal procedures described in pharmacopoeia for the preparation of similar formulations in the various forms above are adopted.

### Example 1

The formulation according to the invention hereafter is in the form of a tablet.

The various components, as indicated below, were weighed for the production of a lot of 30,000 tablets.

The quantities of raw materials necessary for the production of this lot (18,000g inositol; 2g selenium methionine; 5,400g cellulose; 300g GLYCEROL MONO BEHENATE; 450g crospovidone polyplasdone xl; 210g silicon dioxide; 8,638g of anhydrous dibasic calcium phosphate) were first weighed and sieved and then loaded into a mixer of appropriate volume.

After the mixing process, the bulk obtained was loaded in the tablet press for the production of tablets

The tablets thus obtained have a weight of 1,100 mg and contain:

| COMPONENT | mg | % |
|---|---|---|
| INOSITOL | 600.00 | 54.55 |
| SELENIUM METHIONINE | 0.0830 | 0.01 |
| MICROCRISTALLINE CELLULOSE 102 | 180.00 | 16.36 |
| GLYCEROL MONO BEHENATE | 10.00 | 0.91 |
| CROSPOVIDONE POLYPLASDONE XL | 15.00 | 1.36 |
| SILICON DIOXIDE | 7.00 | 0.64 |
| ANHYDROUS DIBASIC CALCIUM PHOSPHATE | 287.92 | 26.17 |

### Activity Test

14 subjects suffering from autoimmune thyroiditis were randomly divided into two groups.

The first group was treated with 83 ug of selenium methionine while the second group was treated with 600 mg of myo-inositol and 83 ug of selenium methionine for six months.

The results are summarised in Table 1.

**TABLE 1**

| Se-methionine | | | | | | | | | | |
|---|---|---|---|---|---|---|---|---|---|---|
| | TS H-pre | TSH-POST | FT3 PRE | FT3-post | FT4 PRE | FT4-post | anti- TPO PRE | anti- TPO POST | anti- TG PRE | anti- TG POST |
| AVER GE | 4.5 | 4.5 | 6.0 | 5.6 | 18.7 | 12.9 | 840.3 | 505.0 | 1216.3 | 737.,8 |
| Δ% | -2% | | 8% | | 31% | | 40% | | 39% | |

| Se-methionine&Myo-inositol | | | | | | | | | | |
|---|---|---|---|---|---|---|---|---|---|---|
| | TS H-pre | TSH-POST | FT3 PRE | FT3-post | FT4 PRE | FT4-post | anti-TPO PRE | anti-TPO POST | anti-TG PRE | anti-TG POST |
| AVER AGE | 4.6 | 3.3 | 6.7 | 5.3 | 18.9 | 11.8 | 838.6 | 405.2 | 992.6 | 525.9 |
| Δ% | 28% | | 20% | | 38% | | 52% | | 47% | |

TSH thyroid stimulating hormone, FT3 free T3 hormone, FT4 free T4 hormone, anti-TPO anti-peroxidase antibodies; anti-TG anti-thyroglobulin antibodies; Δ% change in percentage of the values after treatment

As one can see, the combination therapy is able to reduce the values of TSH by 28% while no change was observed in the group treated with selenium - methionine only.

Moreover, the synergistic action of the two compounds is also inferred on the reduction of FT3 (8% Se-methionine 20% myo-inositol Se-methionine), FT4 (31% Se-methionine 38% myo-inositol Se-methionine). Similar data were observed on the reduction of concentration of anti-TPO antibodies (40% Se-methionine 52% myo-inositol Se-methionine) and anti-TG (39% Se-methionine 47% myo-inositol Se-methionine).

In all cases the differences observed between the two groups were statistically significant.

The formulations are therefore suitable to restore or maintain the euthyroid state, for the treatment of Hashimoto's thyroiditis, to normalize the levels of TSH, T3, T4, FT3, FT4, and to reduce the concentration of anti-peroxidase (anti-TPO globulin and anti-thyroglobulin (anti-TG) individually or taken as a whole.

Moreover the formulation can be successfully used against several autoimmune diseases as it is demonstrated by the results of in-vitro experiments reported in Figures A - E; in this connection it should be noted that the test used is considered predicting for the following autoimmune diseases: Hashmoto Thyroiditis, Grace's diseases, Type 1 diabetes, acute motor axonal neuropathy (AMAN). antiphospholipid syndrome, autoimmune hepatitis, celiac disease, Churg-Strauss syndrome, CREST syndrome, dermatitisherpetiformis, Devic's syndrome, encephalitis, inflammatory myopathy, Isaac's Syndrome, Lambert-Eaton, limbic encephalitis, microscopicpolyangiitis, Miller-Fisher Syndrome, Mixed Connective Tissue Disease, multifocal motor neuropathy with conduction block (MMN), myasthenia gravis, myasthenic syndrome, opsoclonus, polymyositis, primary biliary cirrhosis, primary biliary cirrhosis, Primary Sjögren's syndrome, rheumatoid arthritis, scleromyositis, SLE, Stiff person syndrome, Sydenham's chorea, systemic lupus erythematosus, systemic sclerosis, systemicvasculitides, Wegener's granulomatosis.

In particular, primary cell culture established from healthy tissues are stimulated with interferon gamma (INFg) and tumour necrosis factor alfa (TNFa). Cell stimulation induces the cell culture to produce proinflammatory cytokines such as CXCL 10, CXCL 9 and CXCL11 (cell culture does not produce these cytokines at baseline).

INFg+TNFa treatment occurred alone or on cell culture that were pretreated with Selenomethionine (SelMet) or Myo-inositol (MI) or the combination of both.

Results showed that although with different efficacy, the treatment with MI+SelMet significantly reduces cytokines production (A, C, E).

In another set of experiment MI was substituted with one of its nine stereoisomers: scyllo- inositol.

Surprisingly, the beneficial effect observed in the first set of experiments were no longer observed underlining a myo-inositol specific action.

## Claims

1. A formulation comprising Se-methionine in amounts between 10 and 400 ug and inositol in amounts between 100 and 4000 mg wherein said inositol is selected from: myo-inositol or D-chiro-inositol or mixtures thereof for use in restoring or maintaining the euthyroid state.

2. A formulation for the use according to claim 1 for use in treating Hashimoto's thyroiditis.

3. A formulation for the use according to claims 1 - 2 for use in maintaining or reducing the levels of TSH, T3, T4, FT3, FT4, anti-TPO, anti-TG, intended individually or globally.

4. A formulation comprising Se-methionine in amounts between 10 and 400 ug and inositol in amounts between 100 and 4000 mg wherein said inositol is selected from: myo-inositol or D-chiro-inositol or mixtures thereof for use in the treatment of autoimmune diseases chosen in the group consisting of: Grace's diseases, Type 1 diabetes, acute motor axonal neuropathy (AMAN), antiphospholipid syndrome, autoimmune hepatitis, celiac disease, Churg-Strauss syndrome, CREST syndrome, dermatitisherpetiformis, Devic's syndrome, encephalitis, inflammatory myopathy, Isaac's Syndrome, Lambert-Eaton, limbic encephalitis, microscopicpolyangiitis, Miller-Fisher Syndrome, Mixed Connective Tissue Disease, multifocal motor neuropathy with conduction block (MMN), myasthenia gravis, myasthenic syndrome, opsoclonus, polymyositis, primary biliary cirrhosis, primary biliary cirrhosis, Primary Sjögren's syndrome, rheumatoid arthritis, scleromyositis, SLE, Stiff person syndrome, Sydenham's chorea, systemic lupus erythematosus, systemic sclerosis, systemicvasculitides, Wegener's granulomatosis.

## Patentansprüche

1. Formulierung, umfassend Se-Methionin in Mengen zwischen 10 und 400 ug und Inosit in Mengen zwischen 100 und 4000 mg, wobei der Inosit ausgewählt ist aus: myo-Inosit oder D-chiro-Inosit oder deren Gemischen, zur Verwendung bei der Wiederherstellung oder Aufrechterhaltung des Euthyreotstatus.

2. Formulierung zur Verwendung nach Anspruch 1 zur Verwendung bei der Behandlung von Hashimoto-Thyreoditis.

3. Formulierung zur Verwendung nach den Ansprüchen 1 - 2 zur Verwendung bei der Aufrechterhaltung oder Reduzierung der Niveaus von TSH, T3, T4, FT3, FT4, Anti-TPO, Anti-TG, einzeln oder global vorgesehen.

4. Formulierung, umfassend Se-Methionin in Mengen zwischen 10 und 400 ug und Inosit in Mengen zwischen 100 und 4000 mg, wobei der Inosit ausgewählt ist aus: myo-Inosit oder D-chiro-Inosit oder deren Gemischen, zur Verwendung bei der Behandlung von Autoimmunerkrankungen, gewählt aus der Gruppe, bestehend aus: Grace-Erkrankungen, Diabetes Typ 1, akute motorische axonale Neuropathie (AMAN), Antiphospholipidsyndrom, Autoimmunhepatitis, Zöliakie, Churg-Strauss-Syndrom, CREST-Syndrom, Dermatitis herpetiformis, Devic Syndrom, Enzephalitis, entzündliche Myopathie, Isaacs Syndrom, Lambert-Eaton, limbische Enzephalitis, mikroskopische Polyangiitis, Miller-Fisher-Syndrom, Mixed Connective Tissue Disease, multifokale motorische Neuropathie mit Reizleitungsblockadeg (MMN), Myasthenia gravis, myasthenisches Syndrom, Opsoclonus, Polymyositis, primäre biliäre Zirrhose, primäre biliäre Zirrhose, primäres Sjögren Syndrom, rheumatische Arthritis, Scleromyositis, SLE, Stiff-Person-Syndrom, Sydenhams Chorea, systemischer Lupus erythematodes, systemische Sklerose, systemische Vasculitis, Wegener Granulomatose.

## Revendications

1. Formulation comprenant de la Se-méthionine en des quantités comprises entre 10 et 400 µg et de l'inositol en des quantités comprises entre 100 et 4000 mg, dans laquelle ledit inositol est choisi parmi : myo-inositol ou D-chiro-inositol ou des mélanges de ceux-ci pour restaurer ou maintenir l'état euthyroïdien.

2. Formulation pour l'utilisation selon la revendication 1 pour une utilisation dans le traitement de la thyroïdite de Hashimoto.

3. Formulation pour l'utilisation selon les revendications 1 à 2, destinée à être utilisée pour maintenir ou réduire les niveaux de TSH, T3, T4, FT3, FT4, anti-TPO, anti-TG, prévus individuellement ou globalement.

4. Formulation comprenant de la Se-méthionine en des quantités comprises entre 10 et 400 µg et de l'inositol en des quantités comprises entre 100 et 4000 mg, dans laquelle ledit inositol est choisi parmi : myo-inositol ou D-chiro-inositol ou des mélanges de ceux-ci, pour une utilisation dans le traitement de maladies auto-immunes choisies dans le groupe consistant en : maladies de Grace, diabète de type 1, neuropathie axonale motrice aiguë (AMAN), syndrome des antiphospholipides, hépatite auto-immune, maladie coeliaque, syndrome de Churg-Strauss, syndrome CREST, dermatite herpétiforme, syndrome de Devic, encéphalite, myopathie inflammatoire, syndrome d'Isaac, Lambert-Eaton, encéphalite limbique, polyarthrite microscopique, syndrome de Miller-Fisher, maladie du tissu conjonctif mixte, neuropathie motrice multifocale avec bloc de conduction (MMN), myasthénie grave, syndrome myasthénique, opsoclonus, polymyosite, cirrhose biliaire primitive, cirrhose biliaire primitive, syndrome de Sjögren primaire, polyarthrite rhumatoïde, scléromyosite, LED, syndrome de la personne raide, chorée de Sydenham, lupus systémique érythémateux, sclérose systémique, vascularites systémiques, granulomatose de Wegener.
